Europäisches Patentamt

European Patent Office

Office européen des brevets

(11) Veröffentlichungsnummer: **0 361 082**
**A2**

## (12) EUROPÄISCHE PATENTANMELDUNG

(21) Anmeldenummer: 89115555.8

(22) Anmeldetag: 23.08.89

(51) Int. Cl.5: **C12P 7/18**

(30) Priorität: 01.09.88 DE 3829618
24.07.89 DE 3924423

(43) Veröffentlichungstag der Anmeldung:
04.04.90 Patentblatt 90/14

(84) Benannte Vertragsstaaten:
AT BE CH DE ES FR GB IT LI NL

(71) Anmelder: **Henkel Kommanditgesellschaft auf
Aktien
Henkelstrasse 67
D-4000 Düsseldorf 13(DE)**

Anmelder: **Gesellschaft für
Biotechnologische Forschung mbH (GBF)
Mascheroder Weg 1
D-3300 Braunschweig-Stöckheim(DE)**

(72) Erfinder: **Kretschmann, Josef, Dr.
Friedensstrasse 19
D-4018 Langenfeld(DE)**
Erfinder: **Carduck, Franz-Josef, Dr.
Landstrasse 18
D-5657 Haan(DE)**
Erfinder: **Deckwer, Prof.
Olmsweg 56
D-2900 Oldenburg(DE)**
Erfinder: **Tag, Carmen
Gabelsberger Strasse 6
D-3300 Braunschweig(DE)**
Erfinder: **Biebl, Hanno, Dr.
Tulpenweg 2
D-3340 Wolfenbüttel(DE)**

(54) Fermentative Herstellung von 1,3-Propandiol.

(57) Bei einem Verfahren zur Umwandlung von Glycerin in 1,3-Propandiol durch Mikroorganismen sollte eine großtechnische Lösung gefunden werden. Dies gelang dadurch, daß man einen Mikroorganismenstamm, ausgewählt aus den Gruppen Clostridium, Enterobakterium, Lactobacillus, Bacillus, Citrobacter, Aerobacter, Klebsiella, der auf einer 5 Gew.-%igen Glycerinlösung als einziger Kohlenquelle unter Standardfermentationsbedingungen Glycerin in 1,3-Propandiol mit einer Raumzeitausbeute von mehr als 0,5 g $\cdot$ h$^{-1}$ $\cdot$ l$^{-1}$ umsetzt, und diesen auf Glycerin als einziger Kohlenstoffquelle unter anaeroben Bedingungen zur technischen Umwandlung von Glycerinlösungen mit einer Konzentration von 5 bis 20 Gew.-% Glycerin unter Konstanthaltung des pH-Wertes einsetzt und nach weitgehendem Verbrauch des Glycerins die entstandene Biomasse abtrennt und das Produktgemisch destillativ aufarbeitet.

EP 0 361 082 A2

## Fermentative Herstellung von 1,3-Propandiol

Die Erfindung betrifft ein Verfahren zur technischen Umwandlung von Glycerin in 1,3-Propandiol mit Hilfe von vorzugsweise anaerob wachsenden Mikroorganismen.

Im Zuge der oleochemischen Umwandlung von Fettsäuretriglyceriden in Fettsäurefolgeprodukte wie Fettsäuremethylester oder Fettalkohole fällt aus der Triglyceridspaltung Glycerin an.

Aus der wissenschaftlichen Literatur ist bekannt, daß eine Reihe von Mikroorganismen, und unter diesen insbesondere anaerob lebende Mikroorganismen, in der Lage sind, auf Glycerin zu wachsen und Glycerin dabei in andere Produkte umzuwandeln. Einer der dabei beobachteten Metaboliten ist das 1,3-Propandiol.

1,3-Propandiol ist ein vielseitig verwendbares Diol, das prinzipiell für die gleichen Zwecke wie Ethylenglykol Propylengly kol oder Butandiol eingesetzt werden kann. Bisher wird 1,3-Propandiol durch Addition von Wasser an Acrolein und anschließende Hydrierung gewonnen. Das chemische Verfahren ist jedoch so aufwendig, daß die dabei erhaltenen Verfahrensprodukte für viele mögliche Einsatzzwecke aufgrund ihres hohen Preises nicht geeignet sind.

Die Umwandlung von Glycerin in 1,3-Propandiol durch Mikroorganismen ist in der wissenschaftlichen Literatur an einzelnen Stellen erwähnt. So wird von H. Streekstra et al in Arch. Microbiol. (1987), 147: 268 - 275, die Metabolisierung von Glycerin durch Klebsiella aerogenes NCTC 418 in chemostatisierten Kulturen beschrieben. Wenngleich an dieser Stelle die Einwirkung eines speziellen Klebsiella aerogenes-Stammes auf Glycerin in unterschiedlichen Medien behandelt wird, so finden sich doch keine Hinweise, die es dem Fachmann erlauben, die speziellen Stoffwechselleistungen solcher Organismen für ein technisches Verfahren auszuwerten.

Aufgabe der Erfindung ist es, ein technisch durchführbares Verfahren bereitzustellen, das die Umwandlung von Glycerin in 1,3-Propandiol erlaubt. Dieses Verfahren soll insbesondere von technisch anfallenden Glycerinwässern aus der technischen Verarbeitung von Triglyceriden ausgehen.

Insbesondere ist es Aufgabe der Erfindung, ein derartiges Verfahren zu schaffen, daß von Stämmen ausgeht, deren Fermentation technisch gut zu beherrschen ist und die in der Lage sind, unter Standardfermentationsbedingungen Glycerin in Propandiol mit einer Raumzeitausbeute von mehr als $0,5 \ g \cdot h^{-1} \cdot l^{-1}$ umzusetzen, so beispielsweise von Clostridium butyricum SH 1 (DSM -5431) bzw. AK 1 (DSM 5430), aber auch von Klebsiella pneumoniae- Stämmen, wie K. pneumoniae DSM 2026, aber auch K. oxytoca NRCC 3006.

Gegenstand der Erfindung ist somit ein Verfahren zur Umwandlung von Glycerin in 1,3-Propandiol durch Mikroorganismen, dadurch gekennzeichnet, daß man einen Mikroorganismenstamm, ausgewählt aus den Gruppen Clostridium, Enterobakterium, Lactobacillus, Bacillus, Citrobacter, Aerobacter, Klebsiella, der auf einer 5 Gew.-%igen Glycerinlösung als einziger Kohlenstoffquelle unter Standardfermentationsbedingungen Glycerin in 1,3-Propandiol mit einer Raumzeitausbeute von mehr als $0,5 \ g \cdot h^{-1} \cdot l^{-1}$ umsetzt, und diesen auf Glycerin als einziger Kohlenstoffquelle unter anaeroben Bedingungen zur technischen Umwandlung von Glycerinlösungen mit einer Konzentration von 5 bis 20 Gew.-% Glycerin unter Konstanthaltung des pH-Wertes einsetzt und nach weitgehendem Verbrauch des Glycerins die entstandene Biomasse abtrennt und das Produktgemisch destillativ aufarbeitet.

In einer breiten Ausführungsform geht die Erfindung von dem Grundgedanken aus, daß bei zahlreichen anaerob lebenden Mikroorganismen, wie auch bei einigen aerob lebenden Mikroorganismen, eine Enzymausstattung vorhanden ist, die es erlaubt, Glycerin in 1,3-Propandiol umzuwandeln. Erfindungsgemäß findet daher als erster Schritt eine Selektion von in Frage kommenden Stämmen nach deren Leistungsfähigkeit statt. Dazu werden Stämme aus den Gruppen Clostridium, Enterobakterium, Lactobacillus, Bacillus, Citrobacter, Aerobacter und/oder Klebsiella ausgewählt und auf einer 5 %igen Glycerinlösung als einziger Kohlenstoffquelle unter Standardfermentationsbedingungen wachsen gelassen, wobei man die Raumzeitausbeute bestimmt und solche Stämme auswählt, die eine Raumzeitausbeute über dem genannten Grenzwert erzielen. Unter Standardfermenationsbedingungen ist hier zu verstehen, daß die Mikroorganismen nach Anzucht in einem üblichen Medium, wie dies beispielsweise von den Stammsammlungen vorgeschlagen wird, in ein Salzmedium geführt werden, das auch eine N-Quelle, etwa Hefeextrakt enthält, jedoch als einzige Kohlenstoff- und Energiequelle Glycerin aufweist. Ein geeignetes Standardmedium besonders für Klebsiella findet sich in Beispiel 2 (Fermentermedium). Es kann nach Literaturangaben auf andere Stämme angepaßt werden.

Erfindungsgemäß ist unter technischen Fermentationsbedingungen der pH-Wert konstant zu halten oder zu regeln. Geeignet sind pH-Werte im Bereich von 6 bis 9, insbesondere im Bereich von 6,5 bis 8.

Erfindungsgemäß hat sich gezeigt, daß besonders geeignete Stämme aus einer der folgenden Mikroorganismengruppe ausgewählt werden können: Clostridium perfringens, Clostridium pasteurianum, Clostridium acetobutylicum, Clostridium butylicum, Clostridium butyricum, Clostridium beijerinckii, clostridium kantontoi, Lactobacillus brevis, Lactobacillus buchneri, Citrobacter freundii, Aerobacter aerogenes, Klebsiella pneumoniae, Citrobacter intermedium, Klebsiella aerogenes oder Klebsiella oxytoca. Unter diesen besonders bevorzugt sind Klebsiella pneumoniae DSM 2026, Klebsiella oxytoca NRCC 3006 sowie auch Citrobacter freundii (DSM 30040 oder 30039) und deren Mutanten und Varianten. Weitere geeignete Stämme sind Klebsiella planticola, insbesondere Klebsiella planticola IAM 1133 sowie deren Mutanten und Varianten. Für den Fachmann ist klar, daß die Enzymausstattung der Stämme das entscheidende Kriterium darstellt. Demnach fallen auch alle solche Stämme unter die Erfindung, die die für die Umsetzung von Glycerin zu 1,3-Propandiol wichtige Enzymausstattung durch gentechnologische Arbeitsmethoden übertragen enthalten.

Zur Durchführung des erfindungsgemäßen Verfahrens besonders bevorzugte Stämme sind die Stämme Clostridium butyricum SH 1 (DSM 5431) und AK 1 (DSM 5430). Diese Stämme wurden aus Erd-und Schlammproben in glycerinhaltigen Medien auf Propandiol-Bildung angereichert, nachdem die Proben pasteurisiert wurden. Die Stämme zeigen die folgenden Eigenschaften: Wachstum in PY-Medium ohne Kohlenhydrat, Wachstum in GlucosemineralsalzbiotinMedium, fehlende Gelatine-Hydrolyse, das Zuckerverwertungsspektrum enspricht Clostridium acetobutyricum im Hinblick auf die Verwertung der Zucker Melecitose und Ribose. Die genannten Stämme sind den Klebsiella-Stämmen gegenüber zu bevorzugen, das sie in Risikoklasse 1 (verglichen mit 2) einzustufen sind. Sie zeigen im erfindungsgemäßen Verfahren besonders hohen Glycerinumsatz.

Im Rahmen der Erfindung hat sich gezeigt, daß mit unüblich hohen, von der mikrobiologischen Praxis abweichenden Glycerinkonzentrationen bis 20 Gew.-%, vorzugsweise von 5 bis 15 Gew.-%, insbesondere 10 bis 15 Gew.-% gearbeitet werden kann. Dies ist umso erstaunlicher, da bei derartig hohen Konzentrationen mit Katabolit-Repression zu rechnen gewesen wäre.

Das erfindungsgemäße Verfahren kann nicht nur mit Lösungen von reinem Glycerin durchgeführt werden, sondern allgemein auch mit Glycerinwasser aus der technischen Verarbeitung von Triglyceriden, so beispielsweise aus der Dampfspaltung von Fettsäuretriglyceriden oder aus der Umesterung von Fettsäuretriglyceriden. Derartige Glycerinwässer enthalten als Hauptbestandteil Wasser und Glycerin, weisen jedoch in untergeordnetem Umfang noch Verunreinigungen auf, die aus den Rohstoffen herrühren oder verfahrensbedingt sind.

Im Rahmen der Erfindung hat sich gezeigt, daß Rohglycerinwässer und unter diesen solche aus der Fettspaltung von Talg und anderen Laurinsäure-armen Triglyceriden besonders bevorzugte Ausgangsrohstoffe sind.

Zur Durchführung des erfindungsgemäßen Verfahrens werden die erfindungsgemäß ausgewählten Mikroorganismenstämme vorzugsweise zunächst in einem Vorkulturmedium angezogen. Geeignete Vorkulturmedien sind Salzmedien, die darüber hinaus Glycerin und/oder andere Kohlenstoffquellen wie z.B. Glucose, wie auch Stickstoffquellen, wie beispielsweise Hefeextrakt enthalten.

Vorzugsweise wird die Vorkultur soweit geführt, daß genügend Biomasse zur Beschickung des Fermenters gebildet ist. Die Beschickung des Fermenters erfolgt meist dann, wenn die Kohlenstoffquelle der Vorkultur völlig oder so gut wie völlig aufgebraucht ist.

Die Fermentermedien unterscheiden sich von den Vorkulturmedien darin, daß sie vergleichsweise geringere Mengen an Phosphat, aber auch an Kalium enthalten.

Generell können sowohl die Vorkultur als auch die Fermentermedien die Kationen Natrium, Kalium, Ammonium, Magnesium und Calcium und die Anionen Phosphat, Sulfat, Chlorid enthalten.

Darüber hinaus enthalten die Fermentermedien vorzugsweise noch Spurenelemente, insbesondere Zink, Eisen, Mangan, Kupfer, Kobalt, Bor und/oder Molybdän.

Den genauen Aufbau der Fermentationsmedien kann der Fachmann der einschlägigen Fachliteratur oder auch den Ratschlägen der Stammsammlungen entnehmen. Hinweise finden sich beispielsweise bei C.G.T. Evans, D. Herbert & D.W. Tempest, 1970 "The Continuous Cultivation of Micro-organisms. 2. Construction of a Chemostat", Methods in Microbiology, 2, Academic Press, S. 277 - 327 und bei H. Streekstra, M.J. Teixeira de Mattos, O.M. Neijssel & D.W. Tempest, 1987, "Overflow Metabolism during Anaerobic Growth of Klebsiella aerogenes NCTC 418 on Glycerol and Dihydroxyacetone in Chemostat Culture", Archives of Microbiology, 147, S. 268 - 275.

Beim Einsatz anaerob wachsender Stämme wird die Fermentation in Abwesenheit von Sauerstoff, vorzugsweise unter Stickstoffatmosphäre durchgeführt. Nach einer bevorzugten Ausführungsform wird unter der Atmosphäre der gasförmigen Fermentationsnebenprodukten gearbeitet.

Zur technischen Durchführung des Verfahrens kann man in Vorkulturbehältern ein geeignetes Inokulum einsetzen, das dann mit der Nährlösung vereinigt wird. Die Impfgutmenge (Inokulum) beträgt bezogen auf Gesamtansatz bevorzugt 5 bis 20 Vol.-%, insbesondere 8 bis 15 Vol.-%. Die so entstandene Aufschläm-

EP 0 361 082 A2

mung von Mikroorganismen in der Lösung wird dann in einem Fermentationskessel oder in einer Fermentationskesselkaskade mit Glycerin beaufschlagt. In diesem Fermenter oder in einem weiteren Fermenter der Kaskade können dann weitere Hilfsmittel zugesetzt werden. So können beispielsweise Entschäumer oder auch Filtrationshilfsmittel beigegeben werden. Zur Ernte wird die Kulturbrühe dann entweder kontinuierlich über eine Membranfilterpresse von der Biomasse getrennt oder es wird das Wertprodukt durch einen Filtrationsprozeß, z.B. durch Mikrofiltration kontinuierlich abgetrennt. Nach Auswaschen der gebildeten Biomasse und Vereinigung des Waschwassers mit dem Filtrat kann man in einem weiteren Schritt kontinuierlich oder diskontinuierlich Wasser und Niedrigsieder in einem Verdampfer zumindest teilweise abziehen. Die den Verdampfer verlassenden höher siedenden Anteile, d.h. Gemische der Nährsalze mit den Fermentationsprodukten, können dann beispielsweise auf einen Dünnschichtverdampfer gegeben werden. Das dann erhaltene Kondensat kann durch Rektifikation oder Kurzwegdestillation getrennt werden, woraufhin man 1,3-Propandiol und gewünschtenfalls Nebenprodukte wie 2,3-Butandiol erhält.

Das erfindungsgemäße Verfahren erlaubt es, Glycerin weitgehend der Stoichiometrie entsprechend in 1,3-Propandiol umzusetzen. Die Ausbeute an 1,3-Propandiol beträgt vielfach in etwa 2 mol 1,3-Propandiol aus 3 mol Glycerin. Unter weitgehend wird hier ein Glycerinverbrauch von zumindest 80, vorzugsweise 95 und insbesondere mehr als 99 % verstanden.

Neben 1,3-Propandiol können noch weitere wichtige Wertprodukte entstehen, so z.B. 2,3-Butandiol, Ethanol oder Acetoin, 2,3-Butandiol, Ethanol oder Essigsäure und/oder Milchsäure.

Es hat sich gezeigt, daß die Fermentation bevorzugt in einem Fermenter durchgeführt wird, der mit einem laminar wirkenden Rührwerk mit geringem Schergefälle ausgestattet ist. Derartige Rührwerke sind dem hier betroffenen Fachmann bekannt. Durch die laminare Strömung werden große Schergefälle vermieden, die sich hier negativ auswirken könnten.

Weiterhin ist es bevorzugt, den Leistungseintrag kleinzuhalten. Der Fachmann wird den Leistungseintrag so einstellen, daß bei einer Fermentationstemperatur im Bereich von 27 bis 40°C, bevorzugt 33 bis 38°C, die eingetragene Leistung zur Aufrechterhaltung der Fermentationstemperatur ausreicht und diese nicht erhöht. So soll diese Leistung bevorzugt kleiner 1 KWh/m$^3$ betragen, vorzugsweise zwischen 0,75 und 0,2 KWh/m$^3$.

## Beispiele

Beispiel 1

Flaschenversuche zum Wachstum von Klebsiella pneumoniae auf verschiedenen Glycerinquellen.

Organismus: Klebsielle pneumoniae DSM 2026,

| Medium: | |
|---|---|
| $K_2HPO_4$ | 3,383 g/l |
| $KH_2PO_4$ | 1,293 g/l |
| $NH_4Cl$ | 5,35 g/l |
| $Na_2SO_4 \cdot 10H_2O$ | 0,64 g/l |
| Citronensäure $\cdot$ $H_2O$ | 0,42 g/l |
| $MgCl_2$ | 0,12 g/l |
| $CaCl_2$ | 0,0022 g/l |
| Hefeextrakt | 1,00 g/l |
| Glucose | 3,00 g/l |

Das Medium wurde vor dem Autoklavieren auf pH 7,2 eingestellt.

Kultivierung:

Der Organismus wurde auf dem Salzmedium über Nacht angezogen. Die Inkubation erfolgte bei 37°C unter anaeroben Bedingungen ($N_2$-Gasphase) in 100 ml-Kulturflaschen. Nach der Inkubation wurde jeweils 1 ml Kulturbrühe als Inokulum für die Wachstumsversuche eingesetzt. Es wurden jeweils 2 Parallelversuche bei Einsatz von 3 verschiedenen Glycerinquellen durchgeführt. Als Medium diente o.g. Salzmedium, statt Glucose hier jeweils 20 g/l Glycerin, Glycerin aus der Fettspaltung von Rindertalg mit Heißdampf bzw. Glycerin aus der Fettspaltung von Kokosöl mit Heißdampf.

**T A B E L L E 1 :**

Einsatz von Glycerinen unterschiedlicher Herkunft zur mikrobiellen Herstellung von 1,3-Propandiol (Flaschenversuche mit Klebsiella pneumoniae (DSM 2026)

| Ansatz | max. Zunahme der Opt. | Glycerinausg. konz. (mM) | Glycerinverbr. (%) | Glycerinverbr. (mM) | Ethanol (mM)[2] | Ethanol (%)[1] | Acetat (mM)[2] | Acetat (%)[1] |
|---|---|---|---|---|---|---|---|---|
| Glycerin | 1,18 | 212,5 | 50,8 | 108,2 | 2,9 | 2,8 | 18,0 | 16,9 |
| TALG-Glycerin | 1,26 | 198,4 | 52,6 | 104,4 | 3,6 | 3,5 | 19,6 | 18,8 |
| KOKOS-Glycerin | 1,15 | 208,2 | 54,6 | 114,2 | 3,8 | 3,3 | 19,5 | 17,4 |

Beispiel 2

Batch-Fermentation von Glycerin zu 1,3-Propandiol

Fortsetzung:   T A B E L L E   I

Einsatz von Glycerinen unterschiedlicher Herkunft zur mikrobiellen Herstellung von 1,3-Propandiol
(Flaschenversuche mit Klebsiella pneumoniae (DSM 2026)

| Ansatz | Acetoin $(mM)^2$ | $(\%)^1$ | 2,3-Butandiol $(mM)^2$ | $(\%)^1$ | 1,3-Propandiol $(mM)^2$ | $(\%)^1$ |
|---|---|---|---|---|---|---|
| Glycerin | 0,3 | 0,2 | 12,5 | 11,7 | 81,4 | 76,4 |
| TALG-Glycerin | 2,4 | 2,3 | 8,8 | 8,5 | 82,4 | 79,1 |
| KOKOS-Glycerin | 1,8 | 1,6 | 7,3 | 6,4 | 70,1 | 61,9 |

[1]mmol Produkt/100 mmol metabo. Glycerin

[2]Konzentrationen nach 48 h Inkubation

Organismus: Klebsiella pneumoniae DSM 2026

| Medium: | |
|---|---|
| Vorkulturmedium | |
| $K_2HPO_4$ | 3,383 g/l |
| $KH_2PO_4$ | 1,293 g/l |
| $NH_4Cl$ | 5,35 g/l |
| $Na_2SO_4 \cdot 10H_2O$ | 0,64 g/l |
| Citronensäure $\cdot$ $H_2O$ | 0,42 g/l |
| $MgCl_2$ | 0,12 g/l |
| $CaCl_2$ | 0,0022 g/l |
| Glycerin | 20,00 g/l |
| Hefeextrakt | 1,00 g/l |
| Glucose | 3,00 g/l |

Das Medium wurde vor dem Autoklavieren auf pH 7,2 eingestellt.

| Fermentermedium | |
|---|---|
| $NaH_2PO_4 \cdot 2H_2O$ | 1,56 g/l |
| $NH_4Cl$ | 5,35 g/l |
| KCl | 0,75 g/l |
| $Na_2SO_4 \cdot 10H_2O$ | 0,64 g/l |
| Citronensäure $\cdot$ $H_2O$ | 0,42 g/l |
| $MgCl_2$ | 0,12 g/l |
| $CaCl_2$ | 0,0022 g/l |
| Glycerin | wie in Tabelle angegeben |
| Hefeextrakt | 1,00 g/l |

Spurenelementkonzentration

Beide Medien enthielten folgende Spurenelementkonzentrationen:

| $ZnCl_2$ | 3,42 mg/l |
|---|---|
| $FeCl_3 \cdot 6H_2O$ | 27,00 mg/l |
| $MnCl_2 \cdot 4H_2O$ | 10,00 mg/l |
| $CuCl_2 \cdot 2H_2O$ | 0,85 mg/l |
| $CoCl_2 \cdot 6H_2O$ | 2,38 mg/l |
| $H_3BO_3$ | 0,31 mg/l |
| $Na_2MoO_4$ | 0,02 mg/l |

Antischaummittel

Während der Fermentationen erfolgten Zugaben eines handelsüblichen Antischaummittels.

Kultivierung:

7

Die Bereitstellung des Inokulums (5 %) erfolgte durch ca. 24-stündige Kultivierung bei 37°C unter anaeroben Bedingungen ($N_2$-Gasphase) in Kulturflaschen.

Die Durchführung der Fermentationen erfolgte in einem 4,5-l-Fermenter der Firma SGI (Setric Genie Industriel) mit Temp.-, pH- und U/min-Meß- und Regelsystem der Firma SGI. Die Fermentationen erfolgten bei 37°C, pH 7 (Regelung durch Zugabe von 2,5 n NaOH) unter anaeroben Bedingungen ($N_2$-Begasung) auf einem einfachen Salzmedium (0,1 % Hefeextrakt) mit 50, 100, 150 und 200 g/l Glycerin (p.A.).

Die nachfolgende Tabelle faßt die Ergebnisse der Fermentationen zusammen.

In den Fermentationen mit 50 und 100 g/l Glycerin wurde das Substrat zu 99 % umgesetzt. Bei den Versuchen mit 150 g/l wurden ca. 90 % des Glycerins umgesetzt.

Die höchste 1,3-Propandiolkonzentration konnte bei 150 g/l erreicht werden: 759 mM bzw. 58 g/l, die höchste RZA (Raum-Zeit-Ausbeute) konnte bei einer Glycerinkonzentration von 100 g/l erreicht werden: 2,3 $g \cdot h^{-1} \cdot l^{-1}$.

Ähnliche Batch-Fermentationen wurden mit Stämmen von Citrobacter freundii im 1-l-Maßstab unternommen. Der als besonders erfolgreich erscheinende Stamm Citrobacter freundii DSM 30039 zeigte in Fermentationen mit 50 g/l Glycerin ein günstiges Produktschema.

# TABELLE II

Mikrobielle Umsetzung von Glycerin zu 1,3-Propandiol bei verschiedenen Ausgangskonzentrationen an Glycerin

| Stamm | Glycerin-konz. (g/l) | Glycerin verbrauch (mol%) | Ethanol (mM) | Ethanol (mol%) | Acetat (mM) | Acetat (mol%) | 2,3-Butandiol (mM) | 2,3-Butandiol (mol%) |
|---|---|---|---|---|---|---|---|---|
| K. pneu-moniae DSM 2026 | 50,9 | 99,4 | 22,8 | 4,1 | 87,6 | 15,9 | 3,6 | 0,7 |
| | 96,9 | 99,9 | 65,0 | 6,2 | 188,2 | 17,9 | 10,4 | 1,0 |
| | 154,1 | 88,5 | 110,5 | 7,5 | 205,5 | 13,9 | 10,8 | 0,7 |
| | 201,7 | 53,0 | 91,5 | 7,9 | 108,5 | 9,3 | 1,4 | 0,1 |
| Citro-bacter freundii | 52,9 | 100,0 | 10,7 | 1,8 | 134,1 | 23,1 | --- | --- |

EP 0 361 082 A2

Fortsetzung T A B E L L E II

Mikrobielle Umsetzung von Glycerin zu 1,3-Propandiol bei verschiedenen Ausgangskonzentrationen an Glycerin

| Stamm | D(-)Lactat | | 1,3-Propandiol | | | RZA | Ausbeute |
|---|---|---|---|---|---|---|---|
| | (mM) | (mol%) | (mM) | (mol%) | (g/l) | $(g \cdot h^{-1} \cdot {}^{-1})$ | bezogen auf theor. Max. (66,66%) |
| K. pneumoniae | 32,3 | 5,9 | 294,4 | 53,5 | 22,4 | 1,4 | 80 |
| | 39,7 | 3,8 | 568,4 | 54,0 | 43,2 | 2,3 | 81 |
| | 169,6 | 11,4 | 759,2 | 51,2 | 57,7 | 1,5 | 77 |
| | 122,7 | 10,6 | 419,5 | 36,1 | 31,9 | 0,4 | 54 |
| Citrobacter freundii | 12,7 | 2,1 | 370,0 | 64,0 | 28,2 | 1,2 | 96 |

Beispiel 3

Es wurden weitere Fermentationen unter gleichen Bedingungen, wie in Beispiel 2 angegeben, durchge-

10

führt. Die Fermentation erfolgte bei 37°C, 200 min$^{-1}$ und pH 7 (Regelung durch Zugabe von 5 N Natronlauge) bei einem Arbeitsvolumen von 3 l. Während der Versuche wurden die Reaktoren mit Stickstoff begast, um anaerobe Verhältnisse zu gewährleisten (0,3 NL/min.).

Dabei wurde bei 2 verschiedenen Fermentationen mit Rohglycerin aus Rindertalg gearbeitet, bei denen die Glycerinkonzentration variiert wurde (90 bzw. 140 g/l). In beiden Versuchsansätzen wurde das dargebotene Glycerin zu mehr als 99 % verstoffwechselt. Die erhaltenen Meßergebnisse sind in der folgenden Tabelle aufgeführt:

## TABELLE III

Ergebnisse der Batch-Fermentationen auf Rindertalg:

| Glycerin-quelle | Glycerin-konz. (g/l) | Glycerin-verbrauch (mol%) | Ethanol (mM) | Acetat (mM) | 2,3-Butan-diol (mM) | D(-)-Lactat (mM) |
|---|---|---|---|---|---|---|
| Rindertalg (Henkel) | 89,9 | 99,9 | 69,7 | 168,8 | 15,4 | 43,7 |
| | 139,3 | 99,1 | 174,4 | 186,0 | 11,2 | 184,7 |

EP 0 361 082 A2

Fortsetzung: **T A B E L L E  III**

Ergebnisse der Batch-Fermentationen auf Rindertalg:

| Glycerin-quelle | 1,3-Propandiol (mM) | 1,3-Propandiol (g/l) | RZA $(g \cdot h^{-1} \cdot l^{-1})$ | 1,3 Propandiol-Ausbeute bezogen auf theor. max. (66,66%) |
|---|---|---|---|---|
| Rindertalg (Henkel) | 548,5 | 41,7 | 2,0 | 84 |
| | 807,5 | 61,4 | 1,7 | 81 |

Beispiel 4

Die Versuche wurden mit den Stämmen Clostridium butyricum SH 1 und AK 1 als Batch-Kulturen mit etwa 700 ml Kulturvolumen in einem 1 l-Fermenter (BCC) wiederholt. Das Medium hatte die folgende Zusammensetzung (Angaben pro Liter): $K_2HPO_4$ 1 g, $KH_2PO_4$ 0,5 g, $(NH4)_2SO_4$ 5g/100 g Glycerin, $MgSO_4 \cdot 7H_2O$ 0,2 g, $CaCl_2 \cdot 2H_2O$ 20 mg, $FeSO_4 \cdot 7H_2O$ 5 mg, Hefeextrakt 1 g, Spurenelementlösung, Glycerin wie angegeben. Der pH-Wert wurde in allen Fällen bei pH 7 konstant gehalten, die Temperatur betrug 32 °C. In analoger Weise wurden auch FED-Batch-Kulturen mit Glycerinnachfütterung durchgeführt. Die Ergebnisse sind in den Tabellen 4, 5, 6 und 7 dargestellt.

Tabelle 4

| Vergärung von Glycerin durch Clostridium SH 1 und AK 1 | | | | | | | | | |
|---|---|---|---|---|---|---|---|---|---|
| Stamm | Glycerin | | O.D. 660 nm | "max $h^{-1}$ | Produkt (mmol/l) | | | Glycerin für Produkt (%) | |
| | gegeben | verbraucht | | | 1,3-Propandiol | Essigsäure | Buttersäure | gesamt | Diol |
| | % | mmol/l | mmol/l | | | | | | | |
| SH 1 | 2 | 240 | 240 | 3,4 | 0,56 | 153 | 26 | 18 | 90 | 64 |
| | 5 | 554 | 554 | 6,2 | 0,42 | 381 | 31 | 46 | 91 | 68 |
| | 11 | 1197 | 1087 | 6,2 | 0,27 | 740 | 18 | 128 | 93 | 68 |
| | Fb* | 956 | 896 | 5,2 | - | 550 | 41 | 83 | 85 | 61 |
| AK 1 | 2 | 236 | 229 | 2,9 | 0,56 | 143 | 9 | 26 | 82 | 62 |
| | 5 | 516 | 516 | 4,4 | 0,30 | 388 | 46 | 46 | (102) | (75) |
| | Fb* | 1203 | 1137 | 5,3 | - | 774 | 85 | 96 | 92 | 68 |

*Fed-batch-Kultur

Tabelle 5

| Vergärung von Glyerin durch Clostridium SH 1 (DSM 5431) und AK 1 (DSM 5430) | | | | | | | |
|---|---|---|---|---|---|---|---|
| Stamm | Glycerin | | Dauer h | Produkte (g/l) | | Produktivität g/l-h | |
| | "%" | verbraucht g/l | | 1,3-Propandiol | Essigsäure + Buttersäure | Glycerin | Diol |
| SH 1 | 2 | 22,1 | 9,5 | 11,6 | 2,2 | 2,3 | 1,2 |
| | 5 | 51,0 | 13 | 30,0 | 5,8 | 3,9 | 2,2 |
| | 11 | 100,0 | 29 | 56,2 | 11,2 | 3,4 | 1,9 |
| | FB* | 82,4 | 22 | 41,8 | 9,4 | 3,7 | 1,9 |
| AK 1 | 2 | 21,1 | 9,5 | 10,7 | 2,8 | 2,2 | 1,4 |
| | 5 | 47,5 | 14,5 | 29,5 | 6,8 | 3,3 | 2,0 |
| | FB* | 104,6 | 26 | 58,8 | 13,5 | 4,0 | 2,3 |

* Fed-batch-Kultur

Tabelle 6

| Vergärung von Rohglycerin durch Clostridium SH | | | | |
|---|---|---|---|---|
| Nr. | Glyceringehalt % | Glycerincharge | lag-Phase h | maximale KOH-verbrauchsrate* mmol/l•h |
| 1 | 2 | roh, aus Rindertalg | 2 | 24 |
| 2 | 2 | roh, aus Kokosfett | 9 | 16 |
| 3 | 5 | roh, aus Rindertalg | 4 | 24 |
| 4 | 5 | aus Rindertalg, mit Nr.1 beimpft | 2 | 30 |
| 5 | 5 | roh, aus Kokosfett | 9 | 14 |
| 6 | 5 | aus Kokosfett, mit Nr.2 beimpft | 8 | 8 |

* Die KOH-Vervbrauchsrate ist der Glycerinverbrauchsrate äquivalent

Tabelle 7

| Umsatz von 2 % Glycerin durch Clostiklium SH 1 In Abhänglgkeit von pH-Wert | | | | | |
|---|---|---|---|---|---|
| pH-Wert | Gärungsdauer h | Glycerin umgesetzt g/l | Produkte als Glycerin | | |
| | | | Propandiol % | Essigsäure % | Buttersäure % |
| 5 | (7) | 6 | 62 | 3 | 34 |
| 6 | 12 | 20 | 67 | 7 | 25 |
| 7 | 8 | 20 | 76 | 7 | 17 |
| 7,5 | 12 | 20 | 73 | 12 | 14 |

**Ansprüche**

1. Verfahren zur Umwandlung von Glycerin in 1,3-Propandiol durch Mikroorganismen, dadurch gekennzeichnet, daß man einen Mikroorganismenstamm, ausgewählt aus den Gruppen Clostridium, Enterobakterium, Lactobacillus, Bacillus, Citrobacter, Aerobacter, Klebsiella, der auf einer 5 Gew.-%igen Glycerinlösung als einziger Kohlenquelle unter Standardfermentationsbedingungen Glycerin in 1,3-Propandiol mit einer Raumzeitausbeute von mehr als 0,5 g • h$^{-1}$ • l$^{-1}$ umsetzt, und diesen auf Glycerin als einziger Kohlenstoffquelle unter anaeroben Bedingungen zur technischen Umwandlung von Glycerinlösungen mit einer Konzentration von 5 bis 20 Gew.-% Glycerin unter Konstanthaltung des pH-Wertes einsetzt und nach weitgehendem Verbrauch des Glycerins die entstandene Biomasse abtrennt und das Produktgemisch destillativ aufarbeitet.

2. Verfahren nach Anspruch 1, dadurch gekennzeichnet, daß man als Glycerinlösungen technisches Glycerin, insbesondere technische Glycerinlösungen aus der technischen Verarbeitung von Triglyceriden einsetzt.

3. Verfahren nach einem der Ansprüche 1 oder 2, dadurch gekennzeichnet, daß man als Glycerinlösungen Glycerinlösungen aus der Verseifung und/oder Umesterung von Fetten ohne eine Nachbehandlung der Glycerin-Wasserphase einsetzt.

4. Verfahren nach einem der vorstehenden Ansprüche, dadurch gekennzeichnet, daß man als Glycerinlösungen Glycerinlösungen aus der Verseifung von Fetten mit geringem Laurinsäureanteil einsetzt.

5. Verfahren nach einem der vorstehenden Ansprüche, dadurch gekennzeichnet, daß man mit Glycerinkonzentrationen im Bereich von 5 bis 20 Gew.-%, vorzugsweise 10 bis 15 Gew.-% arbeitet.

6. Verfahren nach einem der vorstehenden Ansprüche, dadurch gekennzeichnet, daß man den pH-Wert

15

in einem Bereich von 6 bis 9, insbesondere 6,5 bis 8 konstant hält.

7. Verfahren nach einem der vorstehenden Ansprüche, dadurch gekennzeichnet, daß man einen Stamm aus einer der folgenden Organismengruppen auswählt: Clostridium perfringens, Clostridium pasteurianum, Clostridium acetobutylicum, Clostridium butylicum, Clostridium butyricum, Clostridium beijerinckii, Clostridium kantontoi, Lactobacillus brevis, Lactobacillus buchneri, Citrobacter freundii, Aerobacter aerogenes, Klebsiella pneumoniae, Citrobacter intermedium, Klebsiella aerogenes oder Klebsiella oxytoca.

8. Verfahren nach einem der vorstehenden Ansprüche, dadurch gekennzeichnet, daß man mit Clostridium butyricum SH 1 (DSM 5431) und/oder Clostridium butyricum AK 1 (DSM 5430) und/oder deren zu 1,3-Propandiol-Bildung befähigten Mutanten oder Varianten arbeitet.

9. Verfahren nach einem der vorstehenden Ansprüche, dadurch gekennzeichnet, daß man mit Klebsiella pneumoniae DSM 2026 und/oder dessen zur 1,3-Propandiol-Bildung befähigten Mutanten oder Varianten arbeitet.

10. Verfahren nach einem der vorstehenden Ansprüche, dadurch gekennzeichnet, daß man mit Klebsiella oxytoca NRCC 3006 und/oder dessen zur Bildung von 1,3-Propandiol befähigten Mutanten und/oder Varianten arbeitet.

11. Verfahren nach einem der vorstehenden Ansprüche, dadurch gekennzeichnet, daß man in Gegenwart eines Salzfermentationsmediums arbeitet, das als N-Quelle Hefeextrakt enthält.

12. Verfahren nach einem der vorstehenden Ansprüche, dadurch gekennzeichnet, daß man in Gegenwart der folgenden Spurenelemente fermentiert: Zink, Eisen, Mangan, Kupfer, Kobalt, Bor und Molybdän.

13. Verfahren nach einem der vorstehenden Ansprüche, dadurch gekennzeichnet, daß eine Fermentationstemperatur von 27 bis 40°C über den Eintrag an Rührenergie aufrechterhält.

14. Verfahren nach einem der vorstehenden Ansprüche, dadurch gekennzeichnet, daß man die entstandene Biomasse mit Hilfe einer Membran-Filterpresse absatzweise oder mit Hilfe der Mikrofiltration kontinuierlich abtrennt.

15. Verfahren nach einem der vorstehenden Ansprüche, dadurch gekennzeichnet, daß man die das Wertprodukt enthaltende Flüssigphase nach dem Abtrennen der Biomasse in einem Verdampfer von einem Teil des Wassers und von Niedrigsiedern befreit und hernach in einem Dünnschichtverdampfer von nichtflüchtigen Komponenten abtrennt, woraufhin man das Kondensat destillativ in 1,3-Propandiol und andere schwerflüchtige Komponenten trennt.

16. Verfahren nach einem der vorstehenden Ansprüche, dadurch gekennzeichnet, daß man die Fermentation in einem Fermenter mit geringem Energieeintrag, vorzugsweise mit einem Energieeintrag kleiner 1,0 KWh/m$^3$ • h, insbesondere zwischen 0,75 und 0,2 KWh/m$^3$ • h, durchführt.

17. Verfahren nach einem der vorstehenden Ansprüche, dadurch gekennzeichnet, daß man die Fermentation einem Fermenter durchführt, der mit einem im laminaren Bereich arbeitenden Rührwerk ausgerüstet ist.

18. Verfahren nach einem der vorstehenden Ansprüche, dadurch gekennzeichnet, daß bei der Fermentation die Impfgutmenge (Inokulum) 5 bis 20 Vol.-%, vorzugsweise 8 bis 10 Vol.-% des Fermentationsansatzes beträgt.